# EUROPÄISCHE PATENTANMELDUNG

(11) **EP 3 660 335 A1**
(43) Veröffentlichungstag der Anmeldung: **03.06.2020**
(21) Anmeldenummer: 18208490.5
(22) Anmeldetag: 27.11.2018
(51) Int. Cl.: F15B 20/00, F15B 13/044, F15B 13/08, F15D 1/12, A61M 1/10

(54) **SCHALTEINHEIT UND PNEUMATISCHES SYSTEM**

(71) Anmelder: Berlin Heart GmbH, 12247 Berlin (DE)
(72) Erfinder: OAKLEY, Matthew, 48231 Warendorf (DE); REITINGER, Thomas, 93413 Cham (DE)
(74) Vertreter: Pfenning, Meinig & Partner mbB

(57) **Zusammenfassung**

Die Erfindung betrifft eine Schalteinheit zum miteinander Verschalten einer ersten pneumatischen Einheit und einer zweiten pneumatischen Einheit eines pneumatischen Systems, wobei die Schalteinheit einen Grundkörper mit einer sich durch den Grundkörper erstreckenden Kanalstruktur, einem ersten und einem zweiten Einlass zum Einleiten eines Drucks in die Kanalstruktur, einem ersten und einem zweiten Auslass zum Ausleiten zumindest eines Teils des Drucks aus der Kanalstruktur sowie einem ersten und zweiten Ventil, wobei der erste Einlass über einen ersten Kanal mit dem ersten Auslass durch Einstellen einer ersten Schaltstellung des ersten Ventils oder über einen zweiten Kanal mit dem zweiten Auslass durch Einstellen einer zweiten Schaltstellung des ersten Ventils in eine Druckaustauschverbindung bringbar ist, und wobei der zweite Einlass über einen dritten Kanal mit dem ersten Auslass durch Einstellen einer ersten Schaltstellung des zweiten Ventils oder über einen vierten Kanal mit dem zweiten Auslass durch Einstellen einer zweiten Schaltstellung des zweiten Ventils in eine Druckaustauschverbindung bringbar ist.

## Beschreibung

Die vorliegende Erfindung betrifft eine Schalteinheit zum miteinander Verschalten zweier pneumatischer Einheiten. Weiterhin betrifft die Erfindung ein pneumatisches System umfassend eine derartige Schalteinheit sowie ein Verfahren zum Betrieb des pneumatischen Systems.

Bekannt sind zum Beispiel Schalteinheiten (auch Umschalteinheiten (USE) genannt) in pneumatischen Blutpumpenantrieben. Die Funktion der Schalteinheit besteht darin, den Betrieb des pneumatischen Systems während eines Ausfalls einer Antriebseinheit (EPE) weiterhin zu gewährleisten. Im Falle des Ausfalls einer pneumatischen Antriebseinheit wird der Luftweg, der zum Antrieb einer oder zweier Blutpumpen notwendig ist, so mittels der Schalteinheit verschaltet, dass im Falle der univentrikulären Nutzung der Luftstrom der zweiten Antriebseinheit auf die eine sich im Einsatz befindliche Blutpumpe und im Falle eines biventrikulären Einsatzes der Luftstrom der verbleibenden intakten Antriebseinheit alternierend auf die beiden Blutpumpen geleitet wird, so dass in beiden Fällen der Patient weiter bis zum Austausch der defekten Antriebseinheit versorgt werden kann.

In der im Stand der Technik bekannten Schalteinheit wird ein Drehkolbenventil eingesetzt. Durch Drehen des Ventilkolbens des Drehkolbenventils können verschiedene Strömungskanalkombinationen freigeschaltet werden. Durch das gezielte Umlenken des Luftstroms können so im biventrikulären Unterstützungsmodus beide Blutpumpen bei Ausfall einer EPE abwechselnd angesteuert werden, bei univentrikulärer Unterstützung gelingt, es den Luftweg so zu verändern, dass eine zweite sich im System befindliche Antriebseinheit die Versorgung der Blutpumpe übernehmen kann.

Die Aufgabe der vorliegenden Erfindung ist es eine alternative Schalteinheit bereitzustellen, die einen kompakten Aufbau aufweist und kurze Schaltzyklen mit einem geringen Druckverlust dauerhaft ermöglicht. Eine weitere Aufgabe der vorliegenden Erfindung ist es, ein pneumatisches System umfassend eine derartige Schalteinheit sowie ein Verfahren zum Betrieb des pneumatischen Systems bereitzustellen.

Die Aufgabe wird durch eine Schalteinheit nach Anspruch 1, ein pneumatisches System nach Anspruch 9 sowie ein Verfahren nach Anspruch 11 gelöst. Vorteilhafte Weiterbildungen der erfindungsgemäßen Schalteinheit sind in den abhängigen Ansprüchen 2 bis 8 aufgeführt. Vorteilhafte Weiterbildungen des erfindungsgemäßen pneumatischen Systems sind im abhängigen Anspruch 10 aufgeführt. Vorteilhafte Weiterbildungen des erfindungsgemäßen Verfahrens sind in den abhängigen Ansprüchen 12 bis 14 aufgeführt.

Eine erfindungsgemäße Schalteinheit zum miteinander Verschalten einer ersten pneumatischen Einheit und einer zweiten pneumatischen Einheit eines pneumatischen Systems umfasst einen Grundkörper mit einer sich durch den Grundkörper erstreckenden Kanalstruktur, einem ersten und einem zweiten Einlass zum Einleiten eines Drucks in die Kanalstruktur, einem ersten und einem zweiten Auslass zum Ausleiten zumindest eines Teils des Drucks aus der Kanalstruktur sowie einem ersten und einem zweiten Ventil.

Dabei ist der erste Einlass über einen ersten Kanal mit dem ersten Auslass durch Einstellen einer ersten Schaltstellung des ersten Ventils oder über einen zweiten Kanal mit dem zweiten Auslass durch Einstellen einer zweiten Schaltstellung des ersten Ventils in eine Druckaustauschverbindung bringbar. Das heißt, zwischen dem ersten Einlass und dem ersten Auslass ist über den ersten Kanal durch Einstellen einer ersten Schaltstellung des ersten Ventils oder zwischen dem ersten Einlass und dem zweiten Auslass über den zweiten Kanal durch Einstellen einer zweiten Schaltstellung des ersten Ventils eine Druckaustauschverbindung herstellbar.

Der zweite Einlass ist über einen dritten Kanal mit dem ersten Auslass durch Einstellen einer ersten Schaltstellung des zweiten Ventils oder über einen vierten Kanal mit dem zweiten Auslass durch Einstellen einer zweiten Schaltstellung des zweiten Ventils in eine Druckaustauschverbindung bringbar. Das heißt, zwischen dem zweiten Einlass und dem ersten Auslass ist über den dritten Kanal durch Einstellen einer ersten Schaltstellung des zweiten Ventils oder zwischen dem zweiten Einlass und dem zweiten Auslass über den vierten Kanal durch Einstellen einer zweiten Schaltstellung des zweiten Ventils eine Druckaustauschverbindung herstellbar.

Unter einer Kanalstruktur werden hier nicht zwingend von einem Einlass zu einem Auslass durchgängige oder offene Kanäle verstanden. Zumindest ein Teil der Kanalstruktur wird durch das Einstellen der ersten oder zweiten Schaltstellung der Ventile verschlossen. Je nach Schaltstellung des ersten Ventils kann der erste Kanal offen, also durchlässig für einen Druckaustausch, und der zweite Kanal verschlossen, also nicht durchlässig für einen Druckaustausch, sein oder umgekehrt. Analog kann je nach Schaltstellung des zweiten Ventils der dritte Kanal offen und der vierte Kanal geschlossen sein oder umgekehrt.

Unter einer Druckaustauschverbindung zwischen einem Einlass und einem Auslass wird hier eine Verbindung zwischen dem Einlass und dem Auslass verstanden, welche einen Druckaustausch zwischen dem Einlass und dem Auslass ermöglicht. Insbesondere kann die Druckaustauschverbindung eine Fluidverbindung umfassen.

Die erfindungsgemäße Schalteinheit weist somit anstelle eines einzigen Drehkolbenventils zwei unabhängige Ventile auf, die so miteinander kombiniert werden können, dass sich eine gewünschte 4/2-Wege-Funktion ergibt. Die Verwendung zweier unabhängiger Ventile ermöglicht kurze Schaltzeiten und führt zu wenig Abrieb in der Schalteinheit.

Das erste und/oder das zweite Ventil kann vorzugsweise ein 3/2-Wege-Ventil sein. Sind sowohl das erste als auch das zweite Ventil 3/2-Wege-Ventile, so ergibt die Kombination beider Ventile in der Schalteinheit eine 4/2-Wege-Funktion.

In einer vorteilhaften Ausgestaltung der Erfindung können das erste und/oder das zweite Ventil als Kolbenventil mit einem Ventilkolben ausgebildet sein, wobei der Ventilkolben innerhalb der Kanalstruktur linear zwischen der ersten und der zweiten Schaltstellung bewegbar ist.

Ferner kann das Kolbenventil eine Magnetspule umfassen, wobei der Ventilkolben durch eine Wechselwirkung mit der Magnetspule bewegbar ist. Insbesondere kann durch Einschalten eines elektrischen Stromes in der Magnetspule der Ventilkolben von der ersten Schaltstellung in die zweite Schaltstellung bewegbar sein.

Das Kolbenventil kann weiterhin eine Rückstellfeder aufweisen, durch die der Ventilkolben von der zweiten Schaltstellung in die erste Schaltstellung bewegbar ist und/oder in der ersten Schaltstellung gehalten werden kann, wenn kein Strom in der Magnetspule eingeschaltet ist.

In einer weiteren Ausgestaltung der Erfindung können das erste und/oder das zweite Ventil einen Sensor, insbesondere einen Hall-Sensor und/oder einen Enkoder, zur Messung einer Kolbenposition des Ventilkolbens des ersten und/oder zweiten Ventils umfassen. Insbesondere kann der Hall-Sensor eingerichtet sein, zu erfassen, ob sich der Ventilkolben des ersten bzw. zweiten Ventils in der ersten oder der zweiten Schaltstellung befindet.

Der Grundkörper der Schalteinheit kann vorzugsweise ein Metall, insbesondere Aluminium, aufweisen oder daraus bestehen.

Die Erfindung schließt auch ein pneumatisches System ein, welches eine erste und eine zweite pneumatische Einheit sowie eine vorstehend beschriebene Schalteinheit umfasst, wobei die erste pneumatische Einheit eine erste Membranfluidpumpe und eine erste Antriebsvorrichtung aufweist, und wobei die zweite pneumatische Einheit eine zweite Membranfluidpumpe und eine zweite Antriebsvorrichtung aufweist, und wobei die erste und/oder die zweite Antriebsvorrichtung eingerichtet sind, die erste und/oder die zweite Membranfluidpumpe anzutreiben.

Insbesondere kann die erste Antriebsvorrichtung über eine erste Einlassdruckleitung mit dem ersten Einlass der Schalteinheit verbunden sein, die erste Membranfluidpumpe über eine erste Auslassdruckleitung mit dem ersten Auslass der Schalteinheit verbunden sein, die zweite Antriebsvorrichtung über eine zweite Einlassdruckleitung mit dem zweiten Einlass der Schalteinheit verbunden sein und die zweite Membranfluidpumpe über eine zweite Auslassdruckleitung mit dem zweiten Auslass der Schalteinheit verbunden sein.

Die erste und/oder die zweite Antriebsvorrichtung können beispielsweise eine Kolbenpumpe oder eine Kompressorpumpe sein.

Die Erfindung schließt auch ein Verfahren zum Betrieb eines vorstehend beschriebenen pneumatischen Systems ein. Insbesondere kann das Verfahren umfassen, dass eine Schaltstellung des ersten oder zweiten Ventils geändert wird, um auf eine andere Antriebsvorrichtung für die erste oder zweite Membranfluidpumpe zu wechseln.

Weiterhin kann das Verfahren umfassen, dass sich der Ventilkolben des ersten Ventils in der ersten Schaltstellung und/oder der Ventilkolben des zweiten Ventils in der zweiten Schaltstellung befindet, sodass die erste Membranfluidpumpe von der ersten Antriebsvorrichtung angetrieben wird und/oder die zweite Membranfluidpumpe von der zweiten Antriebsvorrichtung angetrieben wird.

Weiterhin kann das Verfahren umfassen, dass der Ventilkolben des ersten Ventils in die zweite Schaltstellung gebracht wird und/oder der Ventilkolben des zweiten Ventils in die erste Schaltstellung gebracht wird, sodass die zweite Membranfluidpumpe von der ersten Antriebsvorrichtung angetrieben wird und/oder die erste Membranfluidpumpe von der zweiten Antriebsvorrichtung angetrieben wird.

Die erfindungsgemäße Schalteinheit ermöglicht bei einem geringen Arbeitsdruck der Antriebsvorrichtungen einen geringen Druckverlust. Weiterhin ist die Schalteinheit robust gegenüber Umwelteinflüsse, z. B. Luftfeuchtigkeit oder Öl-Wasser-Emulsion. Dadurch kann auf eine zusätzliche Filterung der Arbeitsluft verzichtet werden, die ansonsten einen zu hohen Druckverlust zur Folge hätte.

Im Folgenden wird ein Ausführungsbeispiel einer erfindungsgemäßen Schalteinheit sowie eines erfindungsgemäßen pneumatischen Systems anhand von Figuren detaillierter beschrieben. Dabei werden verschiedene erfindungswesentliche oder auch vorteilhafte weiterbildende Elemente im Rahmen jeweils eines konkreten Beispiels genannt, wobei auch einzelne dieser Elemente als solche zur Weiterbildung der Erfindung - auch herausgelöst aus dem Kontext des Beispiels und weiterer Merkmale des Beispiels - verwendet werden können. Weiterhin werden in den Figuren für gleiche oder ähnliche Elemente gleiche oder ähnliche Bezugszeichen verwendet, und deren Erläuterung daher teilweise weggelassen.

Es zeigen
- Figur 1: ein pneumatisches Schaltbild eines erfindungsgemäßen pneumatischen Systems,
- Figur 2: eine Perspektivansicht einer erfindungsgemäßen Schalteinheit,
- Figur 3: eine weitere Perspektivansicht der erfindungsgemäßen Schalteinheit.

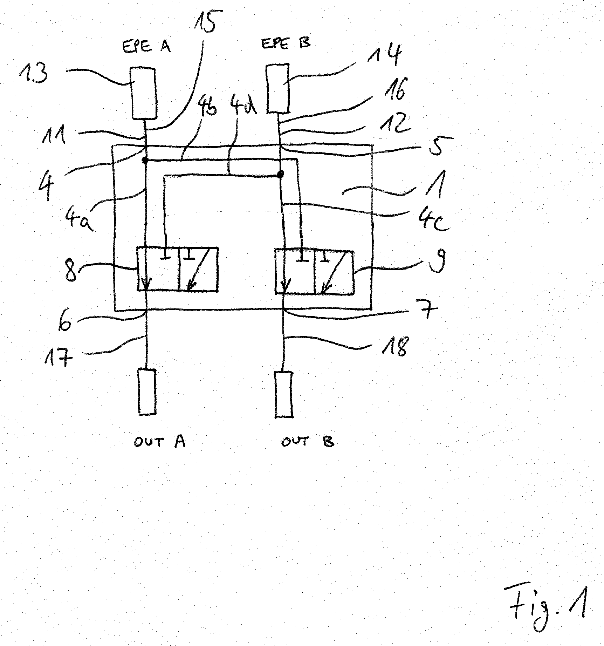

Figur 1 zeigt einen pneumatischen Schaltplan eines erfindungsgemäßen pneumatischen Systems. Das pneumatische System umfasst eine Schalteinheit 1 sowie eine mit der Schalteinheit 1 pneumatisch verbundene erste 11 und zweite pneumatische Einheit 12. Die erste pneumatische Einheit 11 umfasst eine erste Antriebsvorrichtung 13. Die erste Antriebsvorrichtung 13 ist über eine erste Einlassdruckleitung 15 mit einem ersten Einlass 4 der Schalteinheit 1 pneumatisch verbunden. Weiterhin umfasst das pneumatische System eine erste Membranblutpumpe (hier nicht gezeigt), welche über eine erste Auslassdruckleitung 17 mit einem ersten Auslass 6 der Schalteinheit 1 pneumatisch verbunden ist. Die zweite pneumatische Einheit 12 umfasst eine zweite Antriebsvorrichtung 14. Die zweite Antriebsvorrichtung 14 ist über eine zweite Einlassdruckleitung 16 mit einem zweiten Einlass 5 der Schalteinheit 1 pneumatisch verbunden. Weiterhin umfasst das pneumatische System eine zweite Membranblutpumpe (hier nicht gezeigt), welche über eine zweite Auslassdruckleitung 18 mit einem zweiten Auslass 7 der Schalteinheit 1 pneumatisch verbunden ist.

Die Antriebsvorrichtungen 13 und 14 sind eingerichtet, einen vorbestimmten zeitlichen Druckverlauf zum Antreiben der Membranfluidpumpen zu erzeugen. Dieser von den Antriebsvorrichtungen 13 und 14 erzeugte Druckverlauf wird über die Einlassleitungen 15 und 16, die Schalteinheit 1 sowie die Auslassdruckleitungen 17 und 18 an die Membranfluidpumpen übertragen.

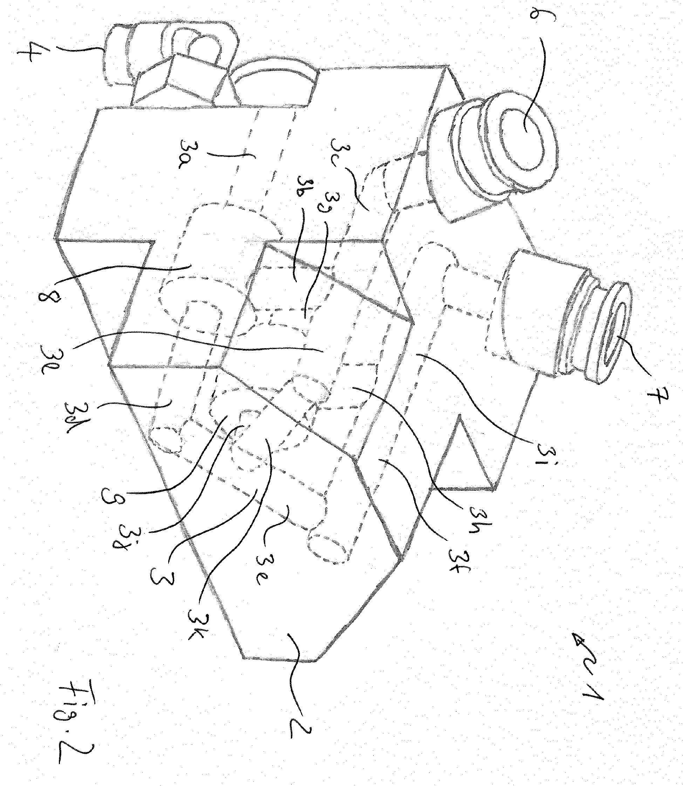

Die Schalteinheit 1 umfasst eine Kanalstruktur 3 (siehe Figur 2) sowie ein erstes Ventil 8 und ein zweites Ventil 9, wobei das erste 8 und das zweite Ventil 9 3/2-Wege-Ventile mit jeweils drei Anschlüssen und zwei Schaltstellungen sind. Das erste und das zweite Ventil 8, 9 ermöglichen für jede Membranblutpumpe zwischen der ersten 13 und der zweiten Antriebsvorrichtung 14 umzuschalten. In einer ersten Schaltstellung des ersten Ventils 8, die in Figur 1 dargestellt ist, befinden sich der erste Einlass 4 und somit die erste Antriebsvorrichtung 13 über eine Druckleitung 4a mit dem ersten Auslass 6 und der ersten Membranblutpumpe in einer Druckaustauschverbindung. Die Druckleitung 4a entspricht damit einem ersten Kanal innerhalb der Kanalstruktur 3 der Schalteinheit 1. In einer zweiten Schaltstellung des ersten Ventils 8, die in Figur 1 nicht dargestellt ist, befinden sich der erste Einlass 4 und somit die erste Antriebsvorrichtung 13 über eine Druckleitung 4d mit dem zweiten Auslass 7 und der zweiten Membranblutpumpe in einer Druckaustauschverbindung. Die Druckleitung 4b entspricht damit einem zweiten Kanal innerhalb der Kanalstruktur 3 der Schalteinheit 1. In einer ersten Schaltstellung des zweiten Ventils 9, die in Figur 1 dargestellt ist, befinden sich der zweite Einlass 5 und somit die zweite Antriebsvorrichtung 14 über eine Druckleitung 4c mit dem zweiten Auslass 7 und der zweiten Membranblutpumpe in einer Druckaustauschverbindung. Die Druckleitung 4c entspricht damit einem dritten Kanal innerhalb der Kanalstruktur 3 der Schalteinheit 1. In einer zweiten Schaltstellung des zweiten Ventils 9, die in Figur 1 nicht dargestellt ist, befinden sich der zweite Einlass 5 und somit die erste Antriebsvorrichtung 14 über eine Druckleitung 4d mit dem zweiten Auslass 7 und der zweiten Membranblutpumpe in einer Druckaustauschverbindung. Die Druckleitung 4d entspricht damit einem vierten Kanal innerhalb der Kanalstruktur 3 der Schalteinheit 1.

Ein Betrieb des pneumatischen Systems ist mit verschiedenen Kombinationen der Schaltstellungen des ersten 8 und zweiten Ventils 9 möglich. Eine erste Kombination sieht vor, dass sich das erste 8 und das zweite Ventil 9 beide in der ersten Schaltstellung befinden, wobei die Druckleitungen 4a und 4c offen sind und die Druckleitungen 4b und 4d geschlossen sind. In diesem Fall wird die erste Membranblutpumpe von der ersten Antriebsvorrichtung 13 und die zweite Membranblutpumpe von der zweiten Antriebsvorrichtung 14 angetrieben. Eine zweite Kombination sieht vor, dass sich das erste und das zweite Ventil 8 und 9 beide in der zweiten Schaltstellung befinden, wobei die Druckleitungen 4b und 4d offen und die Druckleitungen 4a und 4c geschlossen sind. In diesem Fall wird die erste Membranblutpumpe von der zweiten Antriebsvorrichtung 14 und die zweite Membranblutpumpe von der ersten Antriebsvorrichtung 13 angetrieben. Eine dritte Kombination sieht vor, dass sich das erste Ventil 8 in der ersten Schaltstellung und das zweite Ventil 9 in der zweiten Schaltstellung befindet, wobei die Druckleitungen 4a und 4b offen und die Druckleitungen 4c und 4d geschlossen sind. In diesem Fall treibt die erste Antriebsvorrichtung 13 beide Membranblutpumpen an, während die zweite Antriebsvorrichtung 14 nicht im Betrieb ist. Diese Kombination ist somit dann vorteilhaft, wenn die zweite Antriebsvorrichtung 14 ausfällt, da ein biventrikulärer Betrieb des pneumatischen Systems allein mit der ersten Antriebsvorrichtung 13 aufrecht erhalten werden kann. Eine vierte Kombination sieht vor, dass sich das erste Ventil 8 in der zweiten Schaltstellung und das zweite Ventil in der ersten Schaltstellung befindet, wobei die Druckleitungen 4a und 4b geschlossen und die Druckleitungen 4c und 4d offen sind. In diesem Fall treibt die zweite Antriebsvorrichtung 14 beide Membranblutpum-pen an, während die erste Antriebsvorrichtung 13 nicht im Betrieb ist. Diese Kombination ist somit dann vorteilhaft, wenn die erste Antriebsvorrichtung 13 ausfällt, da ein biventrikulärer Betrieb des pneumatischen Systems allein mit der zweiten Antriebsvorrichtung 14 aufrecht erhalten werden kann.

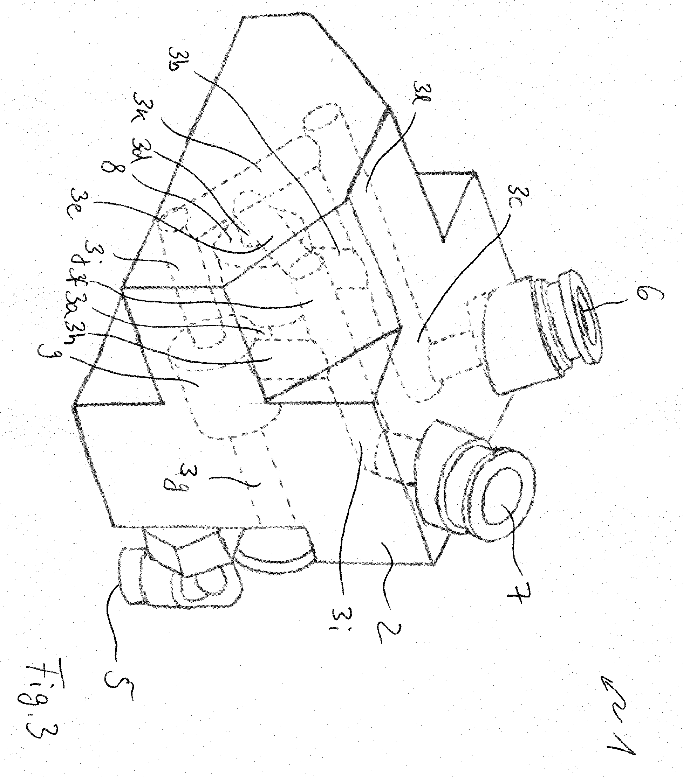

Figuren 2 und 3 zeigen Perspektivansichten einer beispielhaften Ausführungsform einer erfindungsgemäßen Schalteinheit 1 von vorne links und vorne rechts. Die Schalteinheit 1 weist einen im Wesentlichen würfelförmigen Grundkörper 2 mit einer innerhalb des Grundkörpers 2 verlaufenden Kanalstruktur 3 auf. An einer Rückseite des Grundkörpers 2 ragen zwei Anschlüsse aus dem Grundkörper 2 für eine erste Einlassdruckleitung und eine zweite Einlassdruckleitung auf, welche einen ersten Einlass 4 und einen zweiten Einlass 5 bilden. Auf einer an die Rückseite des Grundkörpers 2 angrenzenden Oberseite des Grundkörpers 2 ragen zwei weitere Anschlüsse für Auslassdruckleitungen aus dem Grundkörper heraus. Diese Anschlüsse bilden einen ersten und einen zweiten Auslass 6 und 7. Die Kanalstruktur 3 ist in der Lage einen an einem Einlass 4, 5 anliegenden Druck zu einem Auslass 6, 7 zu übertragen. Innerhalb des Grundkörpers 2 befinden sich weiterhin das erste und das zweite Ventil 8 und 9. Die Kanalstruktur 3 weist mehrere Kanalabschnitte 3a bis 3l, welche mithilfe der Ventile 8 und 9 so kombiniert werden können, dass Druckaustauschverbindungen zwischen mindestens einem der Einlässe 4, 5 und den Auslässen 6 und 7 ermöglicht werden.

Wie bei Figur 1 beschrieben sind vier verschiedene Kombinationen der Schaltstellungen des ersten und zweiten Ventils 8 und 9 denkbar. In der ersten Kombination sind die Druckleitungen 4a und 4c in Figur 1 offen. Die Druckleitungen 4a und 4c entsprechen einem ersten Kanal und einem zweiten Kanal, wobei sich der erste Kanal im Wesentlichen aus Kanalabschnitten 3a, 3b und 3c und der zweite Kanal im Wesentlichen aus Kanalabschnitten 3g, 3h und 3i zusammensetzt. Die Kanalabschnitte 3a und 3i, 3b und 3h sowie 3 c und 3g verlaufen jeweils parallel zueinander. In der zweiten Kombination sind die Druckleitungen 4b und 4d in Figur 1 offen. Die Druckleitungen 4b und 4d entsprechen einem zweiten Kanal und einem vierten Kanal, wobei sich der zweite Kanal aus Kanalabschnitten 3a, 3d, 3e, 3f und 3i und der vierte Kanal aus Kanalabschnitten 3g, 3j, 3k, 3l und 3c zusammensetzen. Die Kanalabschnitte 3d und 3j sowie 3l und 3f verlaufen parallel zueinander, die Kanalabschnitte 3k und 3f verlaufen in parallel zueinander liegenden Ebenen. In der dritten Kombination sind die Druckleitungen 4a und 4b in Figur 1 und somit der erste und zweite Kanal in Figuren 2 und 3 offen. In diesem Fall wird nur ein am ersten Einlass 4 anliegender Druck durch die Kanalstruktur 3 des Grundkörpers 2 zu den Auslässen 6 und 7 übertragen. In der vierten Kombination sind die Druckleitungen 4c und 4d in Figur 1 und somit der dritte und vierte Kanal in den Figuren 2 und 3 offen. In diesem Fall wird nur ein am zweiten Einlass 5 anliegender Druck durch die Kanalstruktur 3 des Grundkörpers 2 zu den Auslässen 6 und 7 übertragen.

Die Ventile 8 und 9 sind zylinderförmig gestaltet und weisen eine Magnetspule sowie eine Rückstellfeder auf, mittels denen ein im Inneren des Ventils entlang der Zylinderachse laufender Ventilkolben bewegbar ist. In der ersten Schaltstellung des ersten 8 und zweiten Ventils 9, in welcher die Druckleitungen 4a und 4c in Figur 1 offen sind, sind das erste Ventil 8 und das zweite Ventil 9 stromlos. Die Ventilkolben werden dabei durch die Rückstellfeder in der ersten Schaltstellung gehalten. In der zweiten Schaltstellung des ersten und zweiten Ventils 8 und 9 fließt ein Strom durch die Magnetspulen der Ventile. Durch die Kraft der bestromten Magnetspulen werden die Ventilkolben unter Überwindung der Kraft der Rückstellfeder in der zweiten Schaltstellung gehalten.

## Patentansprüche

1. Schalteinheit zum miteinander Verschalten einer ersten pneumatischen Einheit und einer zweiten pneumatischen Einheit eines pneumatischen Systems, wobei die Schalteinheit
einen Grundkörper mit einer sich durch den Grundkörper erstreckenden Kanalstruktur, einem ersten und einem zweiten Einlass zum Einleiten eines Drucks in die Kanalstruktur, einem ersten und einem zweiten Auslass zum Ausleiten zumindest eines Teils des Drucks aus der Kanalstruktur sowie einem ersten und zweiten Ventil,
wobei der erste Einlass über einen ersten Kanal mit dem ersten Auslass durch Einstellen einer ersten Schaltstellung des ersten Ventils oder über einen zweiten Kanal mit dem zweiten Auslass durch Einstellen einer zweiten Schaltstellung des ersten Ventils in eine Druckaustauschverbindung bringbar ist,
und wobei der zweite Einlass über einen dritten Kanal mit dem ersten Auslass durch Einstellen einer ersten Schaltstellung des zweiten Ventils oder über einen vierten Kanal mit dem zweiten Auslass durch Einstellen einer zweiten Schaltstellung des zweiten Ventils in eine Druckaustauschverbindung bringbar ist.

2. Schalteinheit nach dem vorhergehenden Anspruch, **dadurch gekennzeichnet, dass** das erste und/oder das zweite Ventil ein 3/2-WegeVentil ist.

3. Schalteinheit nach einem der vorhergehenden Ansprüche, **dadurch gekennzeichnet, dass** das erste und/oder das zweite Ventil als Kolbenventil mit einem Ventilkolben ausgebildet ist, der innerhalb der Kanalstruktur linear zwischen der ersten und der zweiten Schaltstellung bewegbar ist.

4. Schalteinheit nach dem vorhergehenden Anspruch, **dadurch gekennzeichnet, dass** das Kolbenventil eine Magnetspule umfasst, wobei der Ventilkolben durch eine Wechselwirkung mit der Magnetspule bewegbar ist.

5. Schalteinheit nach dem vorhergehenden Anspruch, **dadurch gekennzeichnet, dass** durch Einschalten eines elektrischen Stromes in der Magnetspule der Ventilkolben von der ersten Schaltstellung in die zweite Schaltstellung bewegbar ist.

6. Schalteinheit nach einem der zwei vorhergehenden Ansprüche, **dadurch gekennzeichnet, dass** das Kolbenventil eine Rückstellfeder aufweist, durch die der Ventilkolben von der zweiten Schaltstellung in die erste Schaltstellung bewegbar ist, wenn kein Strom in der Magnetspule eingeschaltet ist.

7. Schalteinheit nach einem der drei vorhergehenden Ansprüche, **dadurch gekennzeichnet, dass** das erste und/oder das zweite Ventil einen Sensor, insbesondere einen Hall-Sensor und/oder einen Enkoder, zur Messung einer Kolbenposition des Ventilkolbens des ersten und/oder zweiten Ventils umfasst.

8. Schalteinheit nach einem der vorhergehenden Ansprüche, **dadurch gekennzeichnet, dass** der Grundkörper ein Metall, insbesondere Aluminium, aufweist oder daraus besteht.

9. Pneumatisches System umfassend eine erste und eine zweite pneumatische Einheit sowie eine Schalteinheit nach einem der vorhergehenden Ansprüche, wobei die erste pneumatische Einheit eine erste Membranfluidpumpe und eine erste Antriebsvorrichtung aufweist, und wobei die zweite pneumatische Einheit eine zweite Membranfluidpumpe und eine zweite Antriebsvorrichtung aufweist, und wobei die erste und/oder die zweite Antriebsvorrichtung eingerichtet sind, die erste und/oder die zweite Membranfluidpumpe anzutreiben.

10. Pneumatisches System nach dem vorhergehenden Anspruch, **dadurch gekennzeichnet, dass** die erste Antriebsvorrichtung über eine erste Einlassdruckleitung mit dem ersten Einlass der Schalteinheit verbunden ist, die erste Membranfluidpumpe über eine erste Auslassdruckleitung mit dem ersten Auslass der Schalteinheit verbunden ist, die zweite Antriebsvorrichtung über eine zweite Einlassdruckleitung mit dem zweiten Einlass der Schalteinheit verbunden ist und die zweite Membranfluidpumpe über eine zweite Auslassdruckleitung mit dem zweiten Auslass der Schalteinheit verbunden ist.

11. Verfahren zum Betrieb eines pneumatischen Systems nach einem der zwei vorhergehenden Ansprüche.

12. Verfahren nach dem vorhergehenden Anspruch, **dadurch gekennzeichnet, dass** eine Schaltstellung des ersten oder zweiten Ventils geändert wird, um auf eine andere Antriebsvorrichtung für die erste oder zweite Membranfluidpumpe zu wechseln.

13. Verfahren nach einem der zwei vorhergehenden Ansprüche, **dadurch gekennzeichnet, dass** sich der Ventilkolben des ersten Ventils in der ersten Schaltstellung und/oder der Ventilkolben des zweiten Ventils in der zweiten Schaltstellung befindet, sodass die erste Membranfluidpumpe von der ersten Antriebsvorrichtung angetrieben wird und/oder die zweite Membranfluidpumpe von der zweiten Antriebsvorrichtung angetrieben wird.

14. Verfahren nach dem vorhergehenden Anspruch, **dadurch gekennzeichnet, dass** der Ventilkolben des ersten Ventils in die zweite Schaltstellung gebracht wird und/oder der Ventilkolben des zweiten Ventils in die erste Schaltstellung gebracht wird, sodass die zweite Membranfluidpumpe von der ersten Antriebsvorrichtung angetrieben wird und/oder die erste Membranfluidpumpe von der zweiten Antriebsvorrichtung angetrieben wird.
